# EUROPEAN PATENT SPECIFICATION

(11) **EP 3 278 814 B1**
(45) Date of publication and mention of the grant of the patent: **18.08.2021**
(21) Application number: 16773084.5
(22) Date of filing: 31.03.2016
(51) Int. Cl.: A61K 38/57, A61K 9/00, A61K 31/727, A61P 7/02

(54) **THERAPEUTIC COMPOSITION CONTAINING ANTITHROMBIN GAMMA**
THERAPEUTISCHE ZUSAMMENSETZUNG MIT ANTITHROMBIN GAMMA
COMPOSITION THÉRAPEUTIQUE CONTENANT DE L'ANTITHROMBINE GAMMA

(30) Priority: 31.03.2015 US 201562140503 P
(43) Date of publication of application: 07.02.2018
(73) Proprietor: Kyowa Kirin Co., Ltd., Tokyo (JP)
(72) Inventor: MUKAI Mayumi, Tokyo (JP)
(74) Representative: Hoffmann Eitle
(86) International application number: PCT/JP2016/060599
(87) International publication number: WO 2016/159204

(56) References cited:
- WO-A1-2005/035563
- WO-A1-2008/100258
- US-B2- 7 691 810
- Anonymous: "A Study of KW-3357 in Congenital Antithrombin Deficiency - Full Text View - ClinicalTrials.gov", , 13 July 2009 (2009-07-13), XP055508833, Retrieved from the Internet: URL:https://clinicaltrials.gov/ct2/show/NC T00938288 [retrieved on 2018-09-21]
- Anonymous: "A Phase 3 Clinical Study of KW-3357 in Patients With DIC - Full Text View - ClinicalTrials.gov", , 29 June 2011 (2011-06-29), XP055508848, Retrieved from the Internet: URL:https://clinicaltrials.gov/ct2/show/NC T01384409?term=KW-3357&rank=2 [retrieved on 2018-09-21]
- JOOST DEJONGH ET AL: "Pharmacokinetics of Recombinant Human Antithrombin in Delivery and Surgery Patients With Hereditary Antithrombin Deficiency", CLINICAL AND APPLIED THROMBOSIS/HEMOSTASIS, vol. 20, no. 4, 11 December 2013 (2013-12-11), pages 355-364, XP55508659, US ISSN: 1076-0296, DOI: 10.1177/1076029613516188
- TOSHIAKI IBA: 'Chiryoyaku Idenshi Kumikae Antithrombin' KYUKYU - SHUCHU CHIRYO vol. 26, no. 5 TO 6, 2014, pages 907 - 910, XP009506376
- AKIRA SATO ET AL.: 'Bio Iyakuhin Kiban Seibi ni Muketa Bio Kozokuhin (Biosimilar) eno Torikumi' PHARM TECH JAPAN vol. 30, no. 7, 2014, pages 1429 - 1434, XP009506358
- TERUHIDE YAMAGUCHI: 'Guideline for follow-on biologics' HUMAN SCIENCE vol. 20, no. 3, 2009, pages 30 - 36, XP009506388
- NEUART JOCHUYO TENPU BUNSHO January 2015, pages 1 - 5, XP009506593

## Description

### Technical Field

The present invention relates to a therapeutic composition comprising antithrombin gamma for blood coagulation such as diffuse intravascular coagulation or disseminated intravascular coagulation involving a decrease of antithrombin, and thrombophilia due to congenital antithrombin deficiency.

### Background Art

Antithrombin is also termed antithrombin III. Human antithrombin is a naturally occurring antithrombin of human and is a glycoprotein consisting of 432 amino acids with a molecular weight of about 59,000 to 65,000, and has three disulfide bonds (Cys8-Cys128, Cys21-Cys95, and Cys247-Cys430) within the molecule (Non-Patent Document 1).

In human antithrombin, N-glycoside-linked sugar chains are attached to four amino acid asparagine numbers 96, 135, 155, and 192 (referred to as N96, N135, N155, and N192, respectively) from the N-terminal.

Human antithrombin in human plasma has two isoforms: α-antithrombin with four N-glycoside-linked sugar chains, and β-antithrombin with three sugar chains and no sugar chain attached to Asn135 (Non-Patent Document 2). 90 to 95% of human antithrombin which exists in human plasma is α-antithrombin, and the remaining 5 to 10% is β-antithrombin.

Complex type N-glycoside-linked sugar chains attached to human antithrombin in human plasma consist of N-acetylglucosamine, sialic acid, galactose, and mannose. One of the characteristics of antithrombin in human plasma is that the sugar chain structure is not fucose-modified.

Human antithrombin is a major anticoagulation factor in blood. By forming conjugates with thrombin, factor X, factor XII, factor IX, or factor XI, human antithrombin inactivates a group of these coagulation factors.

Diffuse intravascular coagulation is also called disseminated intravascular coagulation or DIC for short. DIC is an acquired syndrome characterized by enhanced coagulation that occurs in the blood vessels throughout the body due to multiple factors. Infections, hematopoietic malignancy, and solid cancers are examples of the typical underlying disorders that cause DIC.

The number of DIC patients in Japan is estimated to be 73,000 per year from the nationwide survey conducted in 1997 by Ministry of Health and Welfare, Specific Disease and Hematological Disorder Investigation Research Team. The prognosis of DIC remains poor, with a mortality of 56.0% in 2,193 DIC patients studied by the team (Non-Patent Document 3).

The most common manifestation of DIC is bleeding, which ranges from capillary bleeding, dot hemorrhages, and ecchymotic hemorrhage from an intravenous injection site, to more severe bleeding occurring in the digestive tract, the lungs, or the central nervous system. In DIC, enhanced coagulation develops as microcirculatory vascular occlusion, which may lead to organ failure, and cause clogging of large vessels, and cerebral thrombosis (Non-Patent Document 4).

A goal of DIC treatment is improvement of survival rate in sepsis, and elimination of DIC in diseases such as leukemia. Treatment of DIC begins with treatment of the underlying disorder. Typically, the treatment of the underlying disorder precedes a diagnosis of DIC, and, once diagnosed with DIC, the patient is given a stronger treatment against the underlying disorder, such as administration of antibiotics or antineoplastic drugs (Non-Patent Document 5).

Human antithrombin ranks first in the list of recommended individual drugs in the *Expert Consensus based on Evidence of Treatment of Disseminated Intravascular Coagulation due to Infection* published in 2009 by The Japanese Society on Thrombosis and Hemostasis (Non-Patent Document 6).

Congenital antithrombin deficiency is also called CAD for short. CAD is an autosomal dominant disease characterized by an inherited deficiency of human antithrombin. The genetic abnormality is typically recognized as heterozygous, and the homozygous is considered to be lethal (Non-Patent Document 7). In Japan, the proportion of patient with CAD is estimated to be 0.16% of the population, and is believed to be about the same as that (0.02 to 0.17%) observed in the Europe and America (Non-Patent Document 8).

It is reported that 80 to 90% of CAD patients develops thrombosis by the age of 50 to 60, and the disease often shows first signs of symptoms mainly in the venous system in individuals aged between 10 and 35, particularly after the age of 14 (Non-Patent Document 9). Thrombosis most often occurs in the deep veins in the lower limb, and about 40% of thrombosis is complicated with lung infarction. About 70% of thrombosis is triggered by minor factors that do not usually lead to thrombosis, for example, such as trauma, surgery, pregnancy, and internal use of oral contraceptives.

In an acute phase of thromboembolism, CAD is treated with an antithrombotic therapy and systemic management for cardiovascular failure. For the antithrombotic therapy, thrombolytics is used together with fast-acting anticoagulant heparin. Since the anticoagulant effect of heparin depends on the human antithrombin level in blood, a sufficient anticoagulant effect cannot be expected from heparin in CAD patients, and the treatment requires a supply of a human antithrombin preparation (Non-Patent Document 9).

Since thrombosis is very often recurrent in CAD patients and some patients develop lethal thromboembolism, patients with a history of thrombosis require continuous administration of an oral anticoagulant or an antiplatelet agent. Though a daily supply of a human antithrombin preparation is not needed, a human antithrombin preparation is supplied in high-risk times where causative factors of thrombosis, such as trauma, surgery, pregnancy, and delivery are present (Non-Patent Document 9).

In Japan, preparations containing human antithrombin of human plasma origin (hereinafter, referred to as "plasma-derived human antithrombin") have been approved, and used under the trade name Neuart®, Anthrobin® P, or Kenketu Nonthron® for the indications of "disseminated intravascular coagulation (DIC) involving a decrease of antithrombin III", and "thrombophilia due to congenital antithrombin III deficiency".

However, while the current production of plasma-derived human antithrombin preparations takes safety measures for preventing propagation of infections, it is difficult with the current virus clearance technique to completely deactivate and remove viruses such as human parvovirus B19.

It is also not possible to completely deny the possibility of entry of unknown plasma-derived infectious factors, and the risk of infection by them cannot be eliminated. Further, the fact that plasma-derived human antithrombin preparations are prepared from limited and valuable donated blood poses potential risks in stably supplying human antithrombin preparations, for example, such as a possible reduction of blood donors in the mid to long term.

From the standpoint of stably supply of blood preparations, and self sufficiency of blood preparations from donated blood in Japan, it is stated in Non-Patent Document 10 that "development of alternative blood preparations such as genetically recombinant preparations remains an important challenge." Under these circumstances, a switch is being made to genetically recombinant preparations for the supply of human antithrombin obtained without using human plasma.

There are recent reports concerning genetically recombinant human antithrombin compositions that do not involve binding of fucose to the N-acetylglucosamine at the reducing end of the complex type N-glycoside-linked sugar chains attached to the protein (Patent Documents 1 and 2).

Also known is antithrombin gamma [or antithrombin gamma (genetically recombinant) as it is also called], a genetically recombinant human antithrombin composition that does not involve binding of fucose to the N-acetylglucosamine at the reducing end of the complex type N-glycoside-linked sugar chains attached to the α-protein.

### Related Art

### Patent Document

Patent Document 1: WO2005/035563
Patent Document 2: WO2008/120801

### Non-Patent Document

Non-Patent Document 1: Proc. Natl. Acad. Sci. USA, 80, 1845 (1983)
Non-Patent Document 2: Pathophysiol Haemost Thromb 32, 143 (2002)
Non-Patent Document 3: Masao Nakagawa, Investigation Report on Frequency and Causative Disorder of Disseminated Intravascular Coagulation (DIC) in Japan, Ministry of Health and Welfare, Specific Disease and Hematological Disorder Investigation Research Team, Abnormal Blood Coagulation Session Meeting, Research Report, 1998, 57-64 (1999)
Non-Patent Document 4: Takanori Matsui, Mitsune Tanimoto, 116. In: Tsuguya Fukui, Kiyoshi Kurokawa, Editors, Harrison's Principles of Internal Medicine, 4th Ed., Tokyo: Medical Sciences International Ltd., 855-6 (2013)
Non-Patent Document 5: Hideo Wada, Hideki Nomura, Clinical Thrombosis and Hemostasis - For Doctors in Training, IV 6., Treatment of DIC, Journal of Thrombosis and Hemostasis, 19, 348-52 (2008)
Non-Patent Document 6: Ikuro Maruyama, Yoichi Sakata, Hideo Wada, Hidesaku Asakura, Kenji Okajima, Satoshi Gando, et al., The Japanese Society on Thrombosis and Hemostasis, Scientific Standardization Committee, DIC Society, Expert Consensus based on Evidence of Treatment of Disseminated Intravascular Coagulation due to Infection, Journal of Thrombosis and Hemostasis, 20, 77-113 (2009)
Non-Patent Document 7: Hajime Tsuji, Congenital Antithrombin III (AT III) Deficiency, Journal of Thrombosis and Hemostasis, 12, 74-7 (2001)
Non-Patent Document 8: Toshiyuki Sakata, Hiroshi Matsuo, Akira Okamoto, Yoshiaki Katayama, Toshifumi Mannami, Syunroku Baba, et al., Frequency of Protein C and Antithrombin Deficiency, and their Involvement in Venous thrombosis, [Abstract], Journal of Thrombosis and Hemostasis, 11, 510 (2000)
Non-Patent Document 9: Hajime Tsuji, thrombosis disorders Congenital Thrombosis Disorders Congenital Antithrombin Deficiency/Molecular Abnormality, Nippon Rinsho, Hematological Syndrome, Additional Volume, 2nd Ed., III, 13-6 (2013)
Non-Patent Document 10: Notice on Full Revision of Fundamental Policy for Improvement of Safety of Blood Preparation and Stable Supply of Blood Preparation (Pharmaceutical and Food Safety Bureau Notice 0723, No. 4, July 23, 2013), [Notice on Full Revision of Fundamental Policy for Improvement of Safety of Blood Preparation and Stable Supply of Blood Preparation Pharmaceutical and Food Safety Bureau Notice 0723, No. 4, (July 23, 2013)]

### Disclosure of Invention

### Technical Problem

However, it is not fully clear as to how the effects of antithrombin gamma and plasma-derived human antithrombin differ in the body, and it remains unclear as to the proper administration and dose of antithrombin gamma for the treatment of blood coagulation such as DIC involving a decrease of antithrombin, and thrombophilia attributed to CAD.

It is accordingly an object of the present invention to provide a therapeutic composition comprising antithrombin gamma that can be used in the proper administration and dose for the treatment of blood coagulation such as DIC involving a decrease of antithrombin, and thrombophilia attributed to CAD.

### Means for Solving the Problems

The present inventors conducted intensive studies, and found that a therapeutic composition comprising antithrombin gamma can show efficacy and safety when administered in specific ways in specific doses in the treatment of blood coagulation such as DIC involving a decrease of antithrombin, and thrombophilia attributed to CAD. The present invention was completed on the basis of this finding, as follows.

The present invention pertains to the following (1) to (7).
(1) A therapeutic composition for use in treating diffuse intravascular coagulation or disseminated intravascular coagulation involving a decrease of antithrombin comprising:
   isolated antithrombin gamma in a total daily dose of 36 international units/kg,
   wherein the therapeutic composition is administered by intravenous injection or intravenous drip infusion.
(2) A therapeutic composition for use in treating obstetrical or surgical diffuse intravascular coagulation or disseminated intravascular coagulation comprising:
   isolated antithrombin gamma in a total daily dose of 48 to 72 international units/kg,
   wherein the therapeutic composition is administered as an emergency by intravenous injection or intravenous drip infusion.
(3) The therapeutic composition for use according to (1) or (2),
   wherein the therapeutic composition is administered concurrently with continuous intravenous drip infusion of heparin.
(4) A therapeutic composition for use in treating thrombophilia due to congenital antithrombin deficiency comprising:
   isolated antithrombin gamma in a total daily dose of 24 to 72 international units/kg,
   wherein the therapeutic composition is administered by intravenous injection or intravenous drip infusion.
(5) The therapeutic composition for use according to (4),
   wherein the dose is determined while monitoring an antithrombin activity level.
(6) The therapeutic composition for use according to any one of (1) to (5),
   wherein the therapeutic composition is administered once daily.
(7) The therapeutic composition for use according to any one of (1) to (5),
   wherein the therapeutic composition is administered daily in at least two divided doses.

### Effects of the Invention

A therapeutic composition comprising antithrombin gamma used in accordance with the present invention shows efficacy and safety, and increases the plasma antithrombin activity when properly administered in proper doses in the treatment of disorders such as DIC involving a decrease of antithrombin, obstetrical or surgical DIC, and thrombophilia attributed to CAD.

### Brief Description of Drawings

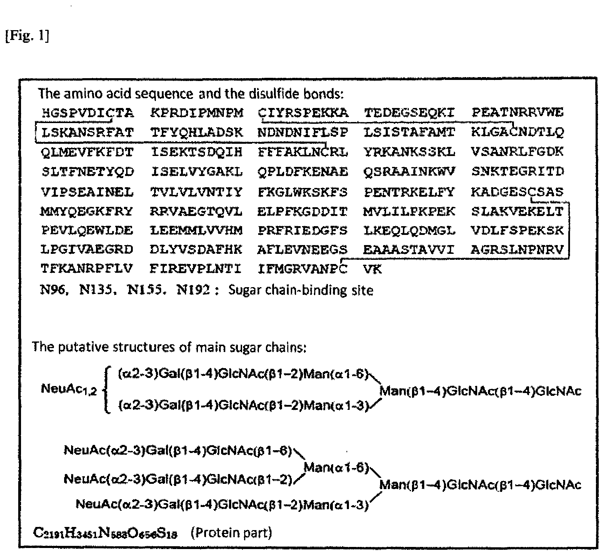

FIG 1 is a diagram showing the amino acid sequence and the disulfide bonds of antithrombin gamma, and the putative structures of main sugar chains.

### Embodiments for Carrying Out the Invention

The following specifically describes the present invention.

FIG. 1 shows the amino acid sequence and the disulfide bonds of antithrombin gamma, and the putative structures of main sugar chains as an example of a specific structure of antithrombin gamma. A specific example of antithrombin gamma is a composition with a molecular weight of about 57,000, termed antithrombin gamma (genetical recombination) according to the Japanese Accepted Names for Pharmaceuticals (also called JAN) (Pharmaceutical and Food Safety Bureau Notice 1126, No.1, Notice on General Designations for Medicine, Registration Number 24-3-B24, November 26, 2014, Director of Inspection Division, Pharmaceutical and Food Safety Bureau, Ministry of Health, Labour and Welfare).

"Isolated antithrombin gamma" of the present invention refers to genetically recombinant human antithrombin, specifically a glycoprotein produced from a Chinese hamster ovary cell (also called "CHO cell") lacking glycoprotein 6-α-L-fucose transferase (or α-1,6-fucosyl transferase as it is also called), and consisting of 432 amino acid residues with four complex type N-glycoside-linked sugar chains .

Isolated antithrombin gamma can be obtained by culturing a transformant created by introducing a DNA encoding human antithrombin into a Chinese hamster ovary cell lacking glycoprotein 6-α-L-fucose transferase, followed by purification of the cell culture medium, using the method described in WO2005/035563 or WO2008/120801.

The bioactivity level of antithrombin gamma is represented by international unit (IU). The bioactivity level of antithrombin gamma can be calculated by, for example, measuring antithrombin activity using international standard for Antithrombin, Concentrate, Human (The 3rd International Standard for Antithrombin, Concentrate, Human, NIBSC code: 06/166), or a standard substance that has been assayed using the International Standard for Antithrombin, Concentrate.

Antithrombin activity can be assayed by a method that quantifies the specific activity of human antithrombin. Specifically, heparin and thrombin are added to human antithrombin of interest to form a conjugate, and after adding a substrate, the absorbance of a color-forming compound is measured as it becomes freed from the substrate in the presence of the residual thrombin whose concentration depends on human antithrombin.

In the present invention, a therapeutic composition comprising antithrombin gamma may be administered once daily, or in divided doses daily. When given in divided doses, the therapeutic composition is administered daily in preferably two or three times.

Intravenous injection (also referred to as intravenous administration or intravenous injection) or intravenous drip infusion (also referred to as intravenous drip infusion, or drip infusion) is examples of a method for administration of the therapeutic composition comprising antithrombin gamma of the present invention.

Since the therapeutic composition comprising antithrombin gamma of the present invention is used as a drug, the therapeutic composition is preferably provided in the form of a composition produced according to a method well known in the field of pharmaceuticals, typically with other components such as one or more pharmaceutically acceptable carriers, additives, and a pH adjuster.

The therapeutic composition comprising antithrombin gamma may be prepared as an injection of a solution form suited for intravenous injection or intravenous drip infusion, by using components such as a carrier comprised of an amino acid, a sugar, a salt, a buffer, or a mixture of these, an additive, and a pH adjuster.

The therapeutic composition comprising antithrombin gamma also may be prepared as an injection of a powder form by freeze drying antithrombin gamma or the therapeutic composition comprising antithrombin gamma according to an ordinary method. When administered as a powdery injection, a powder comprising antithrombin gamma or the therapeutic composition comprising antithrombin gamma is used after being dissolved in injection water, or in a solution such as physiological saline.

In addition to antithrombin gamma, the therapeutic composition comprising antithrombin gamma may contain, for example, preferably sodium citrate, a sodium citrate hydrate, glycine, sodium L-glutamate, D-mannitol, or sodium chloride. More preferably, the therapeutic composition comprising antithrombin gamma may be a composition comprising glycine and a sodium citrate hydrate, or a composition comprising glycine, a sodium citrate hydrate, and sodium chloride. Examples of such compositions include Acoalan®.

The rate of intravenous injection or intravenous drip infusion of the therapeutic composition comprising antithrombin gamma to a patient is not particularly limited in the present invention. For example, the therapeutic composition comprising antithrombin gamma may be slowly administered. The therapeutic composition comprising antithrombin gamma of the present invention may be administered to a patient at the timing when the antithrombin activity level falls below normal, preferably when the antithrombin activity level falls to 70% or less of the normal level.

The antithrombin activity level may be measured by using a commercially available blood testing antithrombin III kit compatible to plasma and whole blood. Examples of such kits include Torinikuromu® AT Xa (available from Kyowa Medex Co., Ltd.), L-System ATIII, Berichrom Antithrombin III Auto B, L-System ATIII (all available from Sysmex), Test Team® Neo ATIII, Test Team® ATIII 2 Kit, Test Team® S ATIII (all available from Sekisui Medical), EVA Test ATIII (available from Nissui Pharmaceutical Co., Ltd.), ATIII Liquid, STA reagent series (all available from Roche Diagnostics), N-Assay TIAATIII, N-Assay LATIII Nittobo (available from Nittobo Medical Co., Ltd.), and Chromorate ATIII (C) II (LSI Medience Corporation) (Pharmaceutical and Food Safety Bureau Notice 0329, No. 10, Notice on Changes to General Designations for Extracorporeal Diagnostic Drugs, March 29, 2011, Director of Inspection Division, Pharmaceutical and Food Safety Bureau, Ministry of Health, Labour and Welfare). The antithrombin activity level may be, for example, plasma antithrombin activity level.

### Therapeutic Composition for DIC

When the therapeutic composition comprising antithrombin gamma is used for treatment of DIC involving a decrease of antithrombin, the therapeutic composition comprising isolated antithrombin gamma in a total daily dose of 36 IU of isolated antithrombin gamma per kilogram body weight of the subject is administered by intravenous injection or intravenous drip infusion.

When the therapeutic composition comprising antithrombin gamma is used for treatment of obstetrical or surgical DIC, the therapeutic composition comprising isolated antithrombin gamma in a total daily dose of 48 to 72 IU of isolated antithrombin gamma per kilogram body weight of the subject is administered by intravenous injection or intravenous drip infusion.

Obstetrical DIC is also called obstetric DIC. Examples of underlying disorders that cause obstetrical DIC include placental abruption, hemorrhagic shock, serious infections, amniotic fluid embolism, eclampsia, serious toxemia of pregnancy, dead fetus syndrome, acute fatty liver of pregnancy, and hydatid mole.

Surgical DIC is also called operative DIC. Examples of underlying disorders that cause surgical DIC include external injury, and bum.

Since obstetrical DIC and surgical DIC are acute, and occur suddenly, the therapeutic composition comprising antithrombin gamma can be administered as an emergency for the treatment of obstetrical DIC or surgical DIC in the present invention.

In the present invention, the therapeutic composition comprising antithrombin gamma may be concurrently administered with continuous intravenous drip infusion of heparin in all DIC, including obstetrical DIC, and surgical DIC.

The heparin used in the present invention may be, for example, a pharmaceutical composition comprising a compound of the heparin family, such as heparin, heparin sodium, heparin calcium, unfractionated heparin, low-molecular-weight heparin, and heparinoids as an active ingredient. Heparin is administered in a daily dose of typically 5,000 to 20,000 units, preferably 10,000 units. The heparin dose is preferably less than 500 units per hour.

### Therapeutic Composition for Thrombophilia Attributed to CAD

When the therapeutic composition comprising antithrombin gamma is used for treatment of thrombophilia attributed to CAD, the therapeutic composition comprising isolated antithrombin gamma in a total daily dose of 24 to 72 IU of isolated antithrombin gamma per kilogram body weight of the subject is administered by intravenous injection or intravenous drip infusion.

In the present invention, it is preferable to administer the therapeutic composition comprising antithrombin gamma in a dose that is determined while monitoring the antithrombin activity level in a treatment of thrombophilia attributed to CAD. The antithrombin activity level may be measured in the manner described above.

In this case, the dose of the therapeutic composition comprising antithrombin gamma is preferably an amount calculated according to the monitored antithrombin activity level so that the antithrombin activity level is confined within the normal range of a healthy individual.

### Examples

The present invention is described below in greater detail using Examples. It should be noted, however, that the following Examples are intended to merely illustrate the present invention, and do not limit the scope of the present invention. The antithrombin gamma preparations of the Examples below represent one form of the therapeutic composition comprising antithrombin gamma described above.

### [Example 1] : Pharmacokinetics Comparative Test of Antithrombin Gamma and Plasma-Derived Human Antithrombin Drugs Administered in Same Amounts

A pharmacokinetics comparative test was conducted under the following conditions according to the phase I clinical trial procedures for antithrombin gamma in Japan. The results are also presented below.

### (1) Subject:

Healthy Adult Male

### (2) Clinical Trial Design:

Randomized, parallel comparison test

### (3) Administration and Dose:

60 IU/kg of an antithrombin gamma preparation, or 60 IU/kg of a plasma-derived human antithrombin preparation was administered once daily for 3 days

### (4) Factors for Choosing Subjects:

1. Individuals who were fully informed of the intent and the contents of the test drug and the clinical trial prior to the clinical trial, and who have submitted a written consent acknowledging that he or she has fully understood the given information, and is willing to be a subject of the clinical trial
2. Japanese males who were at the age of 20 and older and younger than 45 at the time of the consent
3. Individuals who had a BMI of 18.5 or more and less than 25.0 at the time of a pre-trial check

### (5) Factors for Excluding Subjects:

1. Diseased individuals in need of treatment
2. Individuals with a history of medication allergy, or individuals suffering from medication allergy
3. Individuals with a history or a family history of abnormal bleeding or thrombosis
4. Individuals with a history of apparent gastrointestinal hemorrhage (e.g., melena, hematemesis), or individuals suffering from apparent gastrointestinal hemorrhage
5. Alcohol- or drug-dependent individuals
6. Individuals who were not negative for all of the tested items in an infection test (test for HBs antigen, HCV antibody, HIV antibody, and syphilis)
7. Individuals who used a drug (including commercially available drugs and topical agents) within 4 weeks before administration of the test drug
8. Individuals who had other test drugs within 4 months before administration of the test drug
9. Individuals who were admitted to a hospital or had a surgery, or who had more than 200 mL of blood drawn (or donated) within 3 months before administration of the test drug
10. Individuals who have had administration of an antithrombin gamma preparation in the past
11. Individuals who were determined to be inappropriate as subjects of the clinical trial by a chief or an associate doctor conducting the clinical trial

### (6) Results:

Twenty healthy adult males with an age of 20 and older and younger than 45 were given 60 IU/kg of an antithrombin gamma preparation or 60 IU/kg of a plasma-derived human antithrombin (hereinafter, referred to as "pAT") preparation [Neuart® for intravenous injection; hereinafter, referred to as "Neuart®"] by repeated intravenous administration, once daily for 3 days (10 subjects in each group).

For day 1 and 2, the plasma antithrombin activity was measured at three measurement points, before administration and between 1 hour and 10 hours after administration. For day 3, the plasma antithrombin activity was measured at ten measurement points, before administration and between 1 hour and 169 hours after administration. In order to eliminate the influence of the baseline plasma antithrombin activity of the subject, a pharmacokinetics parameter was calculated by subtracting the pre-administration plasma antithrombin activity level (1.08 ± 0.10 IU/mL) of each subject from all post-administration plasma antithrombin activity levels.

Table 1 shows the main evaluation parameters: Cₘₐₓ after the third administration (hereinafter, "C_{max,3rd}"), and AUC, which represents the AUC in a time interval between the third administration (after 48 hours from the first administration) and confirmation of the first subject showing a plasma antithrombin activity below the determination limit (hereinafter, "AUC₄₈₋ₜ").

In Table 1, N represents the number of subjects, C_{max,3rd} represents the highest plasma antithrombin activity after the third administration, AUC₄₈₋ₜ represents the AUC in a time interval between the third administration (after 48 hours from the first administration) and confirmation of the first subject showing a plasma antithrombin activity below the determination limit, and the ratio (%) represents the ratio calculated from the difference in the logarithmically converted mean values of C_{max,3rd} and AUC₄₈₋ₜ of the antithrombin gamma preparation relative to the pAT preparation.

**[Table 1]**

| C_{max,3rd} and AUC₄₈₋ₜ based on plasma antithrombin activity | | | |
|---|---|---|---|
| Parameter | Antithrombin gamma preparation (N = 10) | pAT preparation (N = 10) | Ratio (%) [90% confidence interval] |
| C_{max,3rd} (IU/mL) | 1.67 ± 0.31 | 1.77 ± 0.16 | 92.6 [82.5 to 104.0] |
| AUC₄₈₋ₜ (IU·h/mL) | 58.44 ± 11.72 | 91.44 ± 9.58 | 63.2 [56.0 to 71.3] |

As shown in Table 1, AUC₄₈₋ₜ was lower in the antithrombin gamma preparation group than in the pAT preparation group. From studies of pharmacokinetics data obtained in the test, repeated administration of 72 IU/kg of the antithrombin gamma preparation once daily for 3 days was expected to yield the same pattern of plasma antithrombin activity change as that observed in repeated administration of 60 IU/kg of the pAT preparation once daily for 3 days. The antithrombin gamma preparation, and the pAT preparation tested in the biological equivalence test of Example 2 were therefore set in doses of 72 IU/kg and 60 IU/kg, respectively.

### [Example 2] : Biological Equivalence Test for Antithrombin Gamma and Plasma-Derived Human Antithrombin with Dose of Antithrombin Gamma 1.2 times the Dose of Plasma-Derived Human Antithrombin

A biological equivalence test was conducted under the following conditions according to the phase I clinical trial procedures for antithrombin gamma in Japan. The results are also presented below.

### (1) Subject:

Healthy adult male

### (2) Clinical Trial Design:

Randomized, non-blind parallel comparison test

### (3) Administration and Dose:

72 IU/kg of an antithrombin gamma preparation, or 60 IU/kg of a pAT preparation was administered once daily for 3 days

### (4) Factors for Choosing Subjects:

1. Individuals who have submitted a written consent acknowledging that he or she is willing to participate in the clinical trial
2. Japanese males who were at the age of 20 and older and younger than 45 at the time of the consent
3. Individuals who had a BMI of 18.5 or more and less than 25.0 at the time of a pre-trial check

### (5) Factors for Excluding Subjects:

1. Diseased individuals in need of treatment
2. Individuals with a history of medication allergy, or individuals suffering from medication allergy
3. Individuals with a history or a family history of abnormal bleeding, thrombosis, heart failure, hypokalemia, or long QT syndrome
4. Individuals with a history of apparent gastrointestinal hemorrhage (e.g., melena, hematemesis), or individuals suffering from apparent gastrointestinal hemorrhage
5. Alcohol- or drug-dependent individuals, or individuals who were not negative for all of the tested items in a drug abuse test
6. Individuals who were not negative for all of the tested items in an infection test (test for HBs antigen, HCV antibody, HIV antibody, and syphilis)
7. Individuals who used a drug (including nonprescription drugs, topical agents, vitamin agents, and Chinese medicine) within 4 weeks before administration of the test drug
8. Individuals who participated in a clinical trial of a drug containing new active ingredients, or in a similar test, and were administered with such a drug within 4 months before administration of the test drug
9. Individuals who were admitted to a hospital or had a surgery, or who had more than 200 mL of blood drawn (including blood donation and plasmapheresis) within 3 months before administration of the test drug
10. Individuals who have had administration of an antithrombin gamma preparation in the past
11. Individuals who were determined to be inappropriate as subjects of the clinical trial by a chief or an associate doctor conducting the clinical trial

### (6) Results:

Forty two healthy adult males with an age of 20 and older and younger than 45 were given 72 IU/kg of an antithrombin gamma preparation or 60 IU/kg of a pAT preparation (Neuart®) by repeated intravenous administration, once daily for 3 days (21 subjects in each group). For day 1 and 2, the plasma antithrombin activity was measured at three measurement points, before administration and between 1 hour and 10 hours after administration. For day 3, the plasma antithrombin activity was measured at ten measurement points, before administration and between 1 hour and 169 hours after administration.

In order to eliminate the influence of the baseline plasma antithrombin activity of the subject, a pharmacokinetics parameter was calculated by subtracting the pre-administration plasma antithrombin activity level (1.01 ± 0.09 IU/mL) of each subject from all post-administration plasma antithrombin activity levels. Table 2 shows the main evaluation parameters C_{max,3rd}, and AUC₄₈₋ₜ.

In Table 2, N represents the number of subjects, C_{max,3rd} represents the highest plasma antithrombin activity after the third administration, AUC₄₈₋ₜ represents the ACU in a time interval from the third administration (after 48 hours from the first administration) to the last detection of plasma antithrombin activity, and the ratio (%) represents the ratio calculated from the difference in the logarithmically converted mean values of C_{max,3rd} and AUC₄₈₋ₜ of the antithrombin gamma preparation relative to the pAT preparation.

**[Table 2]**

| C_{max,3rd} and AUC₄₈₋ₜ based on plasma antithrombin activity | | | |
|---|---|---|---|
| Parameter | Antithrombin gamma preparation (72 IU/kg, N = 21) | pAT preparation (60 IU/kg, N = 20) | Ratio (%) [90% confidence interval] |
| C_{max,3rd} (IU/mL) | 2.08 ± 0.17 | 1.98 ± 0.23 | 105.7 [100.3 to 111.3] |
| AUC₄₈₋ₜ (IU·h/mL) | 98.71 ± 13.94 | 98.99 ± 19.82 | 100.5 [91.5 to 110.41 |

As shown in Table 2, 72 IU/kg of the antithrombin gamma preparation, and 60 IU/kg of the pAT preparation were biologically equivalent, and the antithrombin gamma preparation was shown to develop and sustain the same level of efficacy as the pAT preparation when administered in a dose (IU/kg) 1.2 times the dose of the pAT preparation.

### [Example 3]: Comparative Controlled Study of Efficacy and Safety of Antithrombin Gamma and Plasma-Derived Human Antithrombin with Dose of Antithrombin Gamma 1.2 times the Dose of Plasma-Derived Human Antithrombin in Patients with DIC Caused Directly by Infection

A study was conducted for patients diagnosed with DIC caused directly by infection, and that met the acute DIC diagnostic criteria created by the DIC Special Committee of the Japanese Association for Acute Medicine [Journal of the Japanese Association for Acute Medicine, 16, 188-202 (2005); hereinafter referred to as "acute DIC diagnostic criteria"]. The study was conducted under the following conditions according to the phase III clinical trial procedures for antithrombin gamma in Japan. The results are also presented below.

### (1) Subject:

Patients with DIC caused directly by infection

### (2) Clinical Trial Design:

Randomized, non-blind parallel comparison test

### (3) Administration and Dose:

36 IU/kg of an antithrombin gamma preparation, or 30 IU/kg of a pAT preparation was administered once daily for 5 days

### (4) Factors for Choosing Subjects:

1. Patients satisfying the ACCP/SCCM sepsis criteria (at least two of the SIRS items + infection; including severe sepsis, and septic shock)
2. Patients who had 4 points or higher of DIC score in a test conducted at the time of registration according to the acute DIC diagnostic criteria
3. Patients who had an antithrombin activity of 70% or less in a test conducted at the time of registration
4. Patients of either sex who were aged 20 and older at the time of consent
5. Patients who have submitted a written consent by themselves or via a legal representative

### (5) Factors for Excluding Subjects:

1. Patients with a history of severe medication allergy, or patients suffering from severe medication allergy
2. Patients with severe liver damage, such as fulminanthepatitis, and decompensated cirrhosis
3. Patients who are believed to have a limited life span even after elimination of DIC so that there will be only a limited time for administration of the test drug, and sufficient efficacy and safety data would not be obtained
4. Pregnant or nursing patients, or potentially pregnant patients
5. Patients who participated in other clinical trial within 4 months before consent
6. Patients who have had administration of an antithrombin gamma preparation in the past
7. Patients who had a forbidden drug or received a forbidden treatment against the test drug in a time interval from consent to registration
8. Patients who were determined to be inappropriate as subjects of the clinical trial by a chief or an associate doctor conducting the clinical trial

### (6) Results:

A randomized, non-blind parallel comparison test was conducted to investigate the efficacy and safety of the antithrombin gamma preparation. The test was conducted for patients with an age of 20 and older who had a plasma antithrombin activity of 70% or less, and satisfied the ACCP/SCCM sepsis criteria, and who were diagnosed with DIC caused directly by infection according to the acute DIC diagnostic criteria (a DIC score of 4 or more) [target subject size: 200 subjects (100 in each group)].

The antithrombin gamma preparation, or the pAT preparation (Neuart®) were administered by intravenous drip infusion in doses of 36 IU/kg and 30 IU/kg, respectively, together with a compound of the heparin family, once daily for 5 days. However, the antithrombin gamma preparation or the pAT preparation was administered alone when the co-administration of a compound of the heparin family had the risk of causing enhanced bleeding.

The test incorporated 222 subjects (110 subjects in the antithrombin gamma preparation group, and 112 subjects in the pAT preparation group), and all subjects who were given the test drug constituted an ITT (intent-to-treat) population, a group of interest for efficacy analysis.

A compound of the heparin family was administered to a total of 32 subjects out of 109 patients in the antithrombin gamma group, and to 31 subjects out of the 112 patients in the pAT group. A total of 221 subjects (108 subjects in the antithrombin gamma preparation group, and 113 subjects in the pAT preparation group) were analyzed for safety after one of the subjects in the antithrombin gamma preparation group was suspended from the clinical trial before administration of the test drug.

The subject who was originally in the antithrombin gamma preparation group, but was accidentally administered with the pAT preparation was treated as a member of the antithrombin gamma preparation group in the ITT population, and as a member of the pAT preparation group in safety analysis.

The pre-administration plasma antithrombin activity was 54.2% ± 11.5% in the antithrombin gamma preparation group, and 53.2% ± 14.1% in the pAT preparation group. On day 6 post administration, the plasma antithrombin activity was 107.3% ± 26.1% in the antithrombin gamma preparation group, and 115.0% ± 25.3% in the pAT preparation group.

A DIC elimination (defined as having a calculated DIC score of less than 4 according to the acute DIC diagnostic criteria) is a main parameter of efficacy evaluation. The percentage (95% confidence interval) of subjects who showed a DIC elimination on day 6 post administration (or on earlier days when the clinical trial was ended before day 6) was 56.4% [46.6 to 65.8%] in the antithrombin gamma preparation group (62/110 subjects), and 52.7% [43.0 to 62.2%] in the pAT preparation group (59/112 subjects).

With regard to safety, 410 harmful events were observed in 82.4% of the antithrombin gamma preparation group (89/108 subjects), and 494 in 87.6% of the pAT preparation group (99/113 subjects) throughout the course of the test. Forty four cases of side effects were observed in 24 subjects in the antithrombin gamma preparation group, and nineteen cases of side effects were observed in 16 subjects in the pAT preparation group.

As can be seen from these results, 36 IU/kg of the antithrombin gamma preparation group, and 30 IU/kg of the pAT preparation group were shown to provide the same level of plasma antithrombin activity increase, and the same levels of efficacy and safety, regardless of whether a compound of the heparin family were used with the preparations.

### [Example 4] : Test (1) of Efficacy and Safety of Antithrombin Gamma with Dose of Antithrombin Gamma 1.2 times the Dose of Plasma-Derived Human Antithrombin Administered to DIC Patient with Compound of the Heparin Family

A study was conducted for patients diagnosed with DIC or suspected of DIC according to the DIC diagnostic criteria created by the former Ministry of Health and Welfare, Specific Disease and Hematological Disorder Investigation Research Team [Ministry of Health and Welfare, Specific Disease and Hematological Disorder Investigation Research Team, Research Report, 1987, 37-41 (1988); hereinafter, "DIC diagnostic criteria of MHW")]. The study also used a compound of the heparin family, and was conducted under the following conditions according to the phase III clinical trial procedures for antithrombin gamma in Japan. The results are also presented below.

### (1) Subject:

Patients diagnosed with DIC or suspected of DIC according to the DIC diagnostic criteria of MHW

Though the study did not specify underlying disorders, patients having hematopoietic malignancy as an underlying disorder qualified as a subject of the clinical trial.

### (2) Clinical Trial Design:

Multicenter, uncontrolled non-blind study

### (3) Administration and Dose:

The antithrombin gamma preparation was administered in a dose of 36 IU/kg, once daily for 5 days

### (4) Factors for Choosing Subjects:

1. Patients determined as being equivalent of patients diagnosed with DIC or suspected of DIC according to the DIC diagnostic criteria of MHW in a test conducted at the time of registration (a score of 3 or more in a leukemia group, and a score of 6 or more in a non-leukemia group)
   The leukemia group includes patients who have leukemia or related diseases, aplastic anemia, or a prominent reduction of megakaryocytes and a marked platelet decrease after administration of an anti-tumor agent. Patients who fall outside of the leukemia group are in the non-leukemia group.
2. Patients who had an antithrombin activity of 70% or less in a test conducted at the time of registration
3. Patients of either sex who were aged 20 and older at the time of consent
4. Patients who have submitted a written consent by themselves or via a legal representative

### (5) Factors for Excluding Subjects

1. Patients with a history of severe medication allergy, or patients suffering from severe medication allergy
2. Patients with severe liver damage, such as fulminanthepatitis, and decompensated cirrhosis
3. Patients who are believed to have a limited life span even after elimination of DIC so that there will be only a limited time for administration of the test drug, and sufficient efficacy and safety data would not be obtained
4. Pregnant or nursing patients, or potentially pregnant patients
5. Patients who participated in other clinical trial within 4 months before consent
6. Patients who have had administration of an antithrombin gamma preparation in the past
7. Patients who had a forbidden drug or a forbidden treatment against the test drug in a time interval from consent to registration
8. Patients with the risk of enhanced bleeding by co-administration of a compound of the heparin family
9. Patients who were determined to be inappropriate as subjects of the clinical trial by a chief or an associate doctor conducting the clinical trial

### (6) Results:

A non-blind, uncontrolled study was conducted by co-administering a compound of the heparin family to determine the efficacy and safety of the antithrombin gamma preparation. The test was conducted for patients with an age of 20 and older who had a plasma antithrombin activity of 70% or less, and who were diagnosed with DIC or suspected of DIC according to the DIC diagnostic criteria of MHW [a DIC score of 3 or more for patients with leukemia or related diseases, aplastic anemia, or a prominent reduction of megakaryocytes and a marked platelet decrease after administration of an anti-tumor agent (leukemia group), and a DIC score of 6 or more for patients who fell outside of the leukemia group (non-leukemia group); target subject size: at least 10].

The antithrombin gamma preparation was administered by intravenous drip infusion in a dose of 36 IU/kg, together with a compound of the heparin family, once daily for 5 days. In the study, the test drug was administered to 15 subjects (nine in the leukemia group, and six in the non-leukemia group), and the safety and efficacy were analyzed for all subjects. The underlying disorders were acute myelogenous leukemia, myelodysplastic syndrome, non-Hodgkin's lymphoma (two subjects each), and leukemic multiple myeloma, aplastic anemia, and myeloproliferative disease (polycythemia vera) (one subject each) in the leukemia group. In the non-leukemia group, non-Hodgkin's lymphoma (2 subjects), and hemophilia B, non-small cell lung cancer, autoimmune hemolytic anemia, and HIV infection (one subject each) represented underlying disorders.

The plasma antithrombin activity before administration was 54.2% ± 14.1%. The plasma antithrombin activity was 97.5% ± 19.6% on day 6 after the administration of the antithrombin gamma preparation.

The percentage (95% confidence interval) of subjects who showed a DIC elimination on day 6 post administration (or on earlier days when the clinical trial was ended before day 6) which is a main parameter of efficacy evaluation (and is defined as having a calculated DIC score of less than 3 when the underlying disorder is leukemia, and less than 6 when the underlying disorder is non-leukemia according to the DIC diagnostic criteria of MHW) was 40.0% [16.3 to 67.7%] (6/15 subjects).

With regard to safety, 63 harmful events were observed in 80.0% of the subjects (12/15 subjects) throughout the course of the test. The contents of the harmful events observed in two or more subjects are febrile neutropenia (4 cases in 4 subjects), systemicedema (3 cases in 3 subjects), diarrhea (3 cases in 2 subjects), and thrombocytopenia, constipation, vomiting, sepsis, delirium, pruritus, and skin ulcer (2 cases in 2 subjects for each condition). There were no side effects.

As can be seen from these results, 36 IU/kg of the antithrombin gamma preparation was shown to provide the same level of plasma antithrombin activity increase, and the same level of safety as in Examples 3 and 5. However, the percentage of the subjects who showed a DIC elimination was slightly smaller than in Examples 3 and 5, though the preparation had efficacy against DIC. This is probably due to the different methods used for the calculation of DIC score in Example 3 and Example 5. Another possible reason is the larger number of DIC patients with hematopoietic malignancy in Example 4, and the difference in the severity of the underlying disorder.

### [Example 5] : Test (2) of Efficacy and Safety of Antithrombin Gamma with Dose of Antithrombin Gamma 1.2 times the Dose of Plasma-Derived Human Antithrombin Administered to DIC Patient with Compound of the Heparin Family

A study was conducted for patients diagnosed with DIC according to the acute DIC diagnostic criteria. The study also used a compound of the heparin family, and was conducted under the following conditions according to the phase III clinical trial procedures for antithrombin gamma in Japan. The results are also presented below.

### (1) Subject:

Patients diagnosed with DIC according to the acute DIC diagnostic criteria

### (2) Clinical Trial Design:

Multicenter, uncontrolled non-blind test

### (3) Administration and Dose:

The antithrombin gamma preparation was administered in a dose of 36 IU/kg, once daily for 5 days

### (4) Factors for Choosing Subjects:

1. Patients who had a DIC score of 4 or more in a test conducted at the time of registration according to the acute DIC diagnostic criteria
2. Patients who had an antithrombin activity of 70% or less in a test conducted at the time of registration
3. Patients of either sex who were aged 20 and older at the time of consent
4. Patients who have submitted a written consent by themselves or via a legal representative

### (5) Factors for Excluding Subjects:

1. Patients with a history of severe medication allergy, or patients suffering from severe medication allergy
2. Patients with severe liver damage, such as fulminanthepatitis, and decompensated cirrhosis
3. Patients who are believed to have a limited life span even after elimination of DIC so that there will be only a limited time for administration of the test drug, and sufficient efficacy and safety data would not be obtained
4. Pregnant or nursing patients, or potentially pregnant patients
5. Patients who participated in other clinical trial within 4 months before consent
6. Patients who have had administration of an antithrombin gamma preparation in the past
7. Patients who had a forbidden drug or a forbidden treatment against the test drug in a time interval from consent to registration
8. Patients with the risk of enhanced bleeding by co-administration of a compound of the heparin family
9. Patients who were determined to be inappropriate as subjects of the clinical trial by a chief or an associate doctor conducting the clinical trial

### (6) Results

A non-blind, uncontrolled study was conducted by co-administering a compound of the heparin family to determine the efficacy and safety of the antithrombin gamma preparation. The test was conducted for patients with an age of 20 and older who had a plasma antithrombin activity of 70% or less, and who were diagnosed with DIC (a DIC score of 4 or more) according to the acute DIC diagnostic criteria (target subject size: at least 10).

The antithrombin gamma preparation was administered by intravenous drip infusion in a dose of 36 IU/kg, together with a compound of the heparin family, once daily for 5 days. In the study, the antithrombin gamma preparation was administered to 5 subjects, and the safety and efficacy were analyzed for all subjects. The underlying disorders were infections, heat stroke (two subjects each), and acute pancreatitis (one subject).

The plasma antithrombin activity before administration was 53.4% ± 11.1%. The plasma antithrombin activity was 96.8% ± 27.0% on day 6 after the administration of the antithrombin gamma preparation.

The percentage (95% confidence interval) of subjects who showed a DIC elimination on day 6 post administration (or on earlier days when the clinical trial was ended before day 6) which is a main parameter of efficacy evaluation (and is defined as having a calculated DIC score of less than 4 according to the acute DIC diagnostic criteria) was 60.0% [14.7 to 94.7%] (3/5subjects).

With regard to safety, 25 harmful events were observed in 60.0% of the subjects (3/5 subjects) throughout the course of the test. The contents of the harmful events observed in two or more subjects include four cases of atrial fibrillation in 2 subjects. There were no side effects.

As can be seen from these results, 36 IU/kg of the antithrombin gamma preparation was shown to provide the same level of plasma antithrombin activity increase, and the same levels of efficacy and safety as in Example 3, even for DIC patients co-administered with a compound of the heparin family, and having an underlying disorder which is not limited to infection.

While the present invention has been described in detail using a certain embodiment, it will be apparent to a skilled person that various changes and modifications may be made thereto without departing from the scope of the invention. This patent application is based on US provisional patent application No. 62/140,503 filed March 31, 2015.

## Claims

1. (A) A therapeutic composition for use in a method of treating diffuse intravascular coagulation or disseminated intravascular coagulation involving a decrease of antithrombin, the method comprising:
administering isolated antithrombin gamma in a total daily dose of 36 international units/kg,
wherein the therapeutic composition is administered by intravenous injection or intravenous drip infusion,
or
(B) a therapeutic composition for use in a method of treating obstetrical or surgical diffuse intravascular coagulation or disseminated intravascular coagulation, the method comprising:
administering isolated antithrombin gamma in a total daily dose of 48 to 72 international units/kg,
wherein the therapeutic composition is administered as an emergency by intravenous injection or intravenous drip infusion,
or
(C) a therapeutic composition for use in a method of treating thrombophilia due to congenital antithrombin deficiency, the method comprising:
administering isolated antithrombin gamma in a total daily dose of 24 to 72 international units/kg,
wherein the therapeutic composition is administered by intravenous injection or intravenous drip infusion.

2. The therapeutic composition for the use according to claim 1 (A) or 1 (B),
wherein the therapeutic composition is administered concurrently with continuous intravenous drip infusion of heparin.

3. The therapeutic composition for the use according to claim 1 (C),
wherein the dose is determined while monitoring an antithrombin activity level.

4. The therapeutic composition for the use according to any one of claims 1 to 3,
wherein the therapeutic composition is administered once daily.

5. The therapeutic composition for the use according to any one of claims 1 to 3,
wherein the therapeutic composition is administered daily in at least two divided doses.

## Patentansprüche

1. (A) Therapeutische Zusammensetzung zur Verwendung in einem Verfahren zur Behandlung von diffuser intravaskulärer Koagulation oder disseminierter intravaskulärer Koagulation, die eine Abnahme von Antithrombin involviert, wobei das Verfahren umfasst:
Verabreichen von isoliertem Antithrombin-Gamma in einer täglichen Gesamtdosis von 36 internationalen Einheiten/kg,
wobei die therapeutische Zusammensetzung durch intravenöse Injektion oder intravenöse Tropfinfusion verabreicht wird,
oder
(B) therapeutische Zusammensetzung zur Verwendung in einem Verfahren zur Behandlung von geburtshilflicher oder chirurgischer diffuser intravaskulärer Koagulation oder disseminierter intravaskulärer Koagulation, wobei das Verfahren umfasst:
Verabreichen von isoliertem Antithrombin-Gamma in einer täglichen Gesamtdosis von 48 bis 72 internationalen Einheiten/kg,
wobei die therapeutische Zusammensetzung als Notfallmaßnahme durch intravenöse Injektion oder intravenöse Tropfinfusion verabreicht wird,
oder
(C) therapeutische Zusammensetzung zur Verwendung in einem Verfahren zur Behandlung von Thrombophilie aufgrund von angeborenem Antithrombin-Mangel, wobei das Verfahren umfasst:
Verabreichen von isoliertem Antithrombin-Gamma in einer täglichen Gesamtdosis von 24 bis 72 internationalen Einheiten/kg,
wobei die therapeutische Zusammensetzung durch intravenöse Injektion oder intravenöse Tropfinfusion verabreicht wird.

2. Therapeutische Zusammensetzung zur Verwendung gemäß Anspruch 1 (A) oder 1 (B),
wobei die therapeutische Zusammensetzung gleichzeitig mit einer kontinuierlichen intravenösen Tropfinfusion von Heparin verabreicht wird.

3. Therapeutische Zusammensetzung zur Verwendung gemäß Anspruch 1 (C),
wobei die Dosis während der Überwachung eines Antithrombin-Aktivitätsspiegels bestimmt wird.

4. Therapeutische Zusammensetzung zur Verwendung gemäß irgendeinem der Ansprüche 1 bis 3,
wobei die therapeutische Zusammensetzung einmal täglich verabreicht wird.

5. Therapeutische Zusammensetzung zur Verwendung gemäß irgendeinem der Ansprüche 1 bis 3,
wobei die therapeutische Zusammensetzung täglich in mindestens zwei geteilten Dosen verabreicht wird.

## Revendications

1. (A) Composition thérapeutique pour utilisation dans un procédé de traitement de la coagulation intravasculaire diffuse ou de la coagulation intravasculaire disséminée impliquant une diminution de l'antithrombine, le procédé comprenant :
l'administration d'antithrombine gamma isolée à une dose journalière totale de 36 unités internationales/kg,
où la composition thérapeutique est administrée par injection intraveineuse ou perfusion goutte à goutte intraveineuse,
ou
(B) composition thérapeutique pour utilisation dans un procédé de traitement obstétrique ou chirurgical de la coagulation intravasculaire diffuse ou de la coagulation intravasculaire disséminée, le procédé comprenant :
l'administration d'antithrombine gamma isolée à une dose journalière totale de 48 à 72 unités internationales/kg,
où la composition thérapeutique est administrée en urgence par injection intraveineuse ou perfusion goutte à goutte intraveineuse,
ou
(C) composition thérapeutique pour utilisation dans un procédé de traitement de la thrombophilie due à un déficit congénital en antithrombine, le procédé comprenant :
l'administration d'antithrombine gamma isolée à une dose journalière totale de 24 à 72 unités internationales/kg,
où la composition thérapeutique est administrée par injection intraveineuse ou perfusion goutte à goutte intraveineuse.

2. Composition thérapeutique pour l'utilisation selon la revendication 1 (A) ou 1 (B),
où la composition thérapeutique est administrée concomitamment avec une perfusion goutte à goutte intraveineuse continue d'héparine.

3. Composition thérapeutique pour l'utilisation selon la revendication 1 (C),
où la dose est déterminée en surveillant un niveau d'activité antithrombine.

4. Composition thérapeutique pour l'utilisation selon l'une quelconque des revendications 1 à 3,
où la composition thérapeutique est administrée une fois par jour.

5. Composition thérapeutique pour l'utilisation selon l'une quelconque des revendications 1 à 3,
où la composition thérapeutique est administrée chaque jour en au moins deux doses divisées.
